Europäisches Patentamt

European Patent Office

Office européen des brevets

(19)

(11) Veröffentlichungsnummer: **0 037 509 B1**

## EUROPÄISCHE PATENTSCHRIFT

(12)

(45) Veröffentlichungstag der Patentschrift:
13.04.83

(51) Int. Cl.³: **C 07 C 143/60**

(21) Anmeldenummer: 81102194.8

(22) Anmeldetag: 24.03.81

(54) Verfahren zur Herstellung von 1-Naphthylamin-4.7-disulfonsäure.

(30) Priorität: 05.04.80 DE 3013274

(43) Veröffentlichungstag der Anmeldung:
14.10.81 Patentblatt 81/41

(45) Bekanntmachung des Hinweises auf die Patenterteilung:
13.04.83 Patentblatt 83/15

(84) Benannte Vertragsstaaten:
CH DE FR GB IT LI

(56) Entgegenhaltungen:
**DE-A-2 714 031**
**DE-C-41 957**
**BEILSTEINS HANDBUCH DER ORGANISCHEN Chemie, 4. Auflage, Band XIV, Verlag von Julius Springer Berlin, DE.**

(73) Patentinhaber: **BAYER AG, Zentralbereich Patente, Marken und Lizenzen, D-5090 Leverkusen 1, Bayerwerk (DE)**

(72) Erfinder: **Blank, Heinz Ulrich, Dr., Am Geusfelde 35, D-5068 Odenthal (DE)**
Erfinder: **Behre, Horst, Dr., Zur alten Linde 12, D-5068 Odenthal-Eikamp (DE)**
Erfinder: **Linden, Hans Werner, Dr., Heymannstrasse 38, D-5090 Leverkusen (DE)**
Erfinder: **Mentzel, Werner, Dr., Morgengraben 5, D-5000 Koeln 80 (DE)**

## Verfahren zur Herstellung von 1-Naphthylamin-4.7-disulfonsäure

Die Erfindung betrifft ein neues Verfahren zur Herstellung von 1-Naphthylamin-4.7-disulfonsäure.

1-Naphthylamin-4,7-disulfonsäure ist ein wichtiges Zwischenprodukt für die Herstellung von Farbstoffen. Es sind bereits verschiedene Verfahren zur Herstellung von 1-Naphthylamin-4.7-disulfonsäure bekannt. So ist z. B. in der FR-PS 1 490 508 ein Verfahren zur Herstellung von 1-Naphthylamin-4.7-disulfonsäure beschrieben, gemäß dem man 1-Naphthylamin-7-sulfonsäure mit Mangandioxid und Natriumhydrogensulfit sulfoniert. Dieses Verfahren hat jedoch den Nachteil, daß teures Mangandioxid verwendet wird, eine schlechte Raum-Zeit-Ausbeute erzielt wird und große Mengen salzhaltiger Mutterlauge anfallen.

Es kommt daher für ein Verfahren zur Herstellung von 1-Naphthylamin-4.7-disulfonsäure im technischen Maßstab nicht in Betracht.

Von technischem Interesse sind die Verfahren zur Herstellung von 1-Naphthylamin-4.7-disulfonsäure, bei denen die Sulfonierung von 1-Naphthylamin-7-sulfonsäure mit Oleum vorgenommen wird. Solche Verfahren sind z. B. in Beilstein H., XIV, Seite 789, Ullmann, Enzyklopädie der Technischen Chemie, Band 12, Seite 628 (1960); der deutschen Patentanmeldung C 3939 (1891) referiert in Friedländer 3, Seite 432; der GB-PS 15 223; N. Donaldson »The Chemistry and Technology of Naphthalene Compounds« (1958), Seite 203; und der CSSR-PS 129 210 beschrieben.

Gemäß den Angaben in Beilstein soll 1-Naphthylamin-4.7-disulfonsäure beim Eintragen von 1-Naphthylamin-4-sulfonsäure in 25%iges Oleum unterhalb von 30°C und 2−3tägiges Stehenlassen der Sulfonierungsmischung in 70%iger Ausbeute neben 30% 1-Naphthylamin-4.6-disulfonsäure entstehen. Gemäß Ullmann entsteht bei der Sulfonierung von 1-Naphthylamin-7-sulfonsäure mit Schwefelsäuremonohydrat bei 110°C ein Gemisch, in dem 1-Naphthylamin-4.7-disulfonsäure in unbefriedigenden Ausbeuten enthalten ist. Da sich 1-Naphthylamin-4.7-disulfonsäure aus diesen Gemischen nur unter großen Schwierigkeiten abtrennen läßt, haben diese Verfahren zur Herstellung der reinen Verbindungen keine Bedeutung erlangt. Gemäß der deutschen Patentanmeldung C 3939 erfolgt die Herstellung von 1-Naphthylamin-4.7-disulfonsäure durch Sulfonieren von 1-Naphthylamin-7-sulfonsäure bei Temperaturen von 80−140°C in schwach rauchender Schwefelsäure. Nachteilig an diesem Verfahren ist die geringe Ausbeute an 1-Naphthylamin-4.7-disulfonsäure, bedingt durch die Bildung großer Mengen an 1-Naphthylamin-2.4.7-trisulfonsäure. Gemäß dem in der GB-PS 15 223 beschriebenen Verfahren wird 1-Naphthylamin-4.7-disulfonsäure durch Eintragen von 1-Naphthylamin-7-sulfonsäure in 25%iges Oleum und nachfolgendes 3−4stündiges Erwärmen der Sulfonierungsmischung auf 50−60°C erhalten. 1-Naphthylamin-4.7-disulfonsäure wird jedoch nicht isoliert, sondern unmittelbar weiter zu 1-Naphthylamin-2.4.7-trisulfonsäure weitersulfoniert. Die Nacharbeitung dieses Herstellungsverfahrens ergab, daß sich bei diesem beträchtliche Mengen an unerwünschten Nebenprodukten bilden wie 1-Naphthylamin-2.4.7-trisulfonsäure und sich nur unbefriedigende Ausbeuten an der gewünschten 1-Naphthylamin-4.7-disulfonsäure erzielen lassen. Nach dem in Donaldson »The Chemistry and Technology of Naphthalene Compounds« beschriebenen Verfahren wird 1-Naphthylamin-4.7-disulfonsäure durch Sulfonieren von in Monohydrat vorgelegter 1-Naphthylamin-7-sulfonsäure mittels 65%igem Oleum bei Temperaturen unterhalb von 35°C hergestellt. Die Ausbeuten dieses Verfahrens sind mit 92−94% der Theorie angegeben. Nachteilig an diesem Verfahren ist jedoch der hohe Überschuß an Sulfonierungsmittel (Oleum) und die durch diesen Überschuß bedingte große Menge anfallender Dünnsäure bei der Aufarbeitung der Sulfonierungsgemische. In der CSSR-PS 129 210 wird 1-Naphthylamin-4.7-disulfonsäure durch Sulfonieren des N.N′-disubstituierten Harnstoff-Derivates der 1-Naphthylamin-7-sulfonsäure hergestellt. Die Ausbeute beträgt lediglich 75−80% der Theorie. Durch die Einführung der Schutzgruppe (Carbamidogruppe) wird das Verfahren zudem unnötig kompliziert.

Es wurde nun gefunden, daß man 1-Naphthylamin-4.7-disulfonsäure in ausgezeichneten Ausbeuten, hoher Reinheit, ohne die Verwendung eines großen Überschusses an Sulfonierungsmittel herstellen kann, wenn man die Sulfonierung von 1-Naphthylamin-7-sulfonsäure unter bestimmten Bedingungen vornimmt. Die bestimmten Bedingungen bestehen darin, daß man a) das Sulfonierungsagens Oleum zu in Schwefelsäure gelöster bzw. suspendierter 1-Naphthylamin-7-sulfonsäure bzw. gleichzeitig mit 1-Naphthylamin-7-sulfonsäure in vorgelegte Schwefelsäure gibt und b) ein solches Molverhältnis Schwefeltrioxid: 1-Naphthylamin-7-sulfonsäure anwendet, daß 1,2 bis 3 Mol Schwefeltrioxid auf 1 Mol 1-Naphthylamin-7-sulfonsäure entfallen.

Die Erfindung betrifft daher ein Verfahren zur Herstellung von 1-Naphthylamin-4.7-disulfonsäure durch Sulfonieren von 1-Naphthylamin-7-sulfonsäure mit Oleum und Aufarbeiten des Sulfonierungsgemisches durch Eintragen in Wasser und Isolieren der 1-Naphthylamin-4.7-disulfonsäure, das dadurch gekennzeichnet ist, daß man das Oleum bei einer Temperatur von 10−70°C, vorzugsweise 20−50°C entweder gleichzeitig mit 1-Naphthylamin-7-sulfonsäure zu vorgelegter Schwefelsäure gibt, oder, vorzugsweise, zu in Schwefelsäure gelöster, bzw. suspendierter 1-Naphthylamin-7-sulfonsäure gibt und ein solches Molverhältnis Schwefeltrioxid zu 1-Naphthylamin-7-sulfonsäure anwendet, das 1,2 bis 3 Mol, vorzugsweise 1,75 bis 2,5 Mol Schwefeltrioxid auf 1 Mol 1-Naphthylamin-7-sulfonsäure entfallen.

Die Sulfonierung wird bei Temperaturen von 10–70°C, vorzugsweise bei 20–50°C vorgenommen.

Das in dem erfindungsgemäßen Verfahren zu verwendende Oleum kann 20 bis 100 Gew.-% freies Schwefeltrioxid enthalten. Vorzugsweise wird ein Oleum verwendet, das 50 bis 80 Gew.-% freies Schwefeltrioxid enthält.

Nach beendeter Zugabe des Oleums läßt man das Reaktionsgemisch bei Temperaturen von 10 bis 60°C, vorzugsweise 20 bis 40°C unter Rühren ausreagieren. Die Ausreagierzeit liegt zwischen 2 bis 32, vorzugsweise 4 bis 16 Stunden.

Zum Lösen bzw. Suspendieren der 1-Naphthylamin-7-sulfonsäure wird vorzugsweise 80 bis 100%ige, insbesondere 96 bis 100%ige Schwefelsäure verwendet. Die Menge an zum Lösen bzw. Suspendieren verwendeter Schwefelsäure wird vorteilhaft so bemessen, daß auf 1 Mol 1-Naphthylamin-7-sulfonsäure 6 bis 8 Mol Schwefelsäure entfallen.

Wird zum Lösen bzw. Suspendieren wasserhaltige Schwefelsäure verwendet und/oder keine trockene, sondern feuchte 1-Naphthylamin-7-sulfonsäure eingesetzt, so ist für die Sulfonierung über die Menge von 1,2 bis 3 Mol Schwefeltrioxid je Mol 1-Naphthylamin-7-sulfonsäure hinaus so viel Schwefeltrioxid (in Form von Oleum) zuzusetzen, wie zum Binden des durch die Reagenzien eingebrachten Wassers als Schwefelsäure erforderlich sind.

Die 1-Naphthylamin-4.7-disulfonsäure wird aus dem Sulfonierungsgemisch in an sich bekannter Weise durch Eintragen des Sulfonierungsgemisches in Wasser und Abfiltrieren der auskristallisierten Säure bzw. nach Aussalzen, z. B. mit Kochsalz, des Mononatriumsalzes, isoliert.

## Beispiel 1

In einem mit Rührer, Tropftrichter, Innenthermometer und Trockenrohr versehenen Reaktionsgefäß werden 700 g 100%ige Schwefelsäure vorgelegt. In diese werden 225 g (1 Mol) 1-Naphthylamin-7-sulfonsäure (99%ig) bei einer Temperatur von höchstens 40°C eingetragen. Dann werden bei 30°C innerhalb von 30 Minuten 246 g 65%iges Oleum (=2 Mol $SO_3$) zugetropft. Anschließend wird 8 Stunden bei 30°C nachgerührt.

Dann wird das Sulfonierungsgemisch so schnell in etwa 1650 g Wasser eingetragen, daß die entstehende Suspension jederzeit gut rührbar bleibt. Die Temperatur steigt dabei auf etwa 95–100°C. Die Suspension wird anschließend auf 110°C erwärmt und 1 Stunde auf dieser Temperatur gehalten. Anschließend wird je eine weitere Stunde bei 90°C und 100°C gerührt und anschließend langsam auf 20°C abgekühlt. Der Niederschlag wird abgesaugt und zweimal mit wenig 50%iger Schwefelsäure gewaschen.

Es werden 450 g 1-Naphthylamin-4.7-disulfonsäure in Form eines schwach grau gefärbten schwefelsäurefeuchten Produktes erhalten. Die Ausbeute an 1-Naphthylamin-4.7-disulfonsäure beträgt 91,2% der Theorie bezogen auf eingesetzte 1-Naphthylamin-7-sulfonsäure. Die Hochdruck-Flüssigkeitschromatographie des schwefelsäurefeuchten Produktes ergab folgende Zusammensetzung des Produktes:

| | |
|---|---|
| 1-Naphthylamin-4.7-disulfonsäure | 61,5 Gew.-% |
| 1-Naphthylamin-7-sulfonsäure | 0,05 Gew.-% |
| 1-Naphthylamin-2.4.7-trisulfonsäure | 0,05 Gew.-% |
| unbekannte Verunreinigungen (5 Komponenten) | 0,7 Gew.-% |
| Rest bis 100%: $H_2O/H_2SO_4$ | |

## Beispiel 2

Es wird wie in Beispiel 1 beschrieben sulfoniert, nur werden statt 246 g 65%igem Oleum nur 215 g 65%iges Oleum (=1,75 Mol $SO_3$) verwendet. Außerdem wird 16 Stunden bei 30°C nachgerührt.

Es werden 475 g 1-Naphthylamin-4.7-disulfonsäure in Form eines schwach grauen, schwefelsäurefeuchten Produktes erhalten. Die Ausbeute an 1-Naphthylamin-4.7-disulfonsäure beträgt 91,6% der Theorie, bezogen auf eingesetzte 1-Naphthylamin-7-sulfonsäure.

Die Hochdruck-Flüssigkeitschromatographie des schwefelsäurefeuchten Produktes ergab folgende Zusammensetzung des Produktes:

| | |
|---|---|
| 1-Naphthylamin-4.7-disulfonsäure | 58,9 Gew.-% |
| 1-Naphthylamin-7-sulfonsäure | 0,05 Gew.-% |
| 1-Naphthylamin-2.4.7-trisulfonsäure | 0,05 Gew.-% |
| unbekannte Verunreinigungen (6 Komponenten) | 0,7 Gew.-% |
| Rest bis 100%: $H_2O/H_2SO_4$ | |

0 037 509

Beispiel 3

Es wird wie in Beispiel 1 beschrieben sulfoniert, nur werden statt 246 g 65%igem Oleums nur 185 g 65%iges Oleum ( = 1,5 Mol SO₃) verwendet und 24 Stunden bei 30° C nachgerührt.

Es werden 505 g 1-Naphthylamin-4.7-disulfonsäure in Form eines schwach grauen, schwefelsäurefeuchten Produktes erhalten. Die Ausbeute an 1-Naphthylamin-4.7-disulfonsäure beträgt 90,9% der Theorie, bezogen auf eingesetzte 1-Naphthylamin-7-sulfonsäure. Die Hochdruck-Flüssigkeitschromatographie des schwefelsäurefeuchten Produktes ergab folgende Zusammensetzung des Produktes:

| | |
|---|---|
| 1-Naphthylamin-4.7-disulfonsäure | 54,6 Gew.-% |
| 1-Naphthylamin-7-sulfonsäure | 0,05 Gew.-% |
| 1-Naphthylamin-2.4.7-trisulfonsäure | 0,05 Gew.-% |
| unbekannte Verunreinigungen (5-Komponenten) | 1,5 Gew.-% |
| Rest bis 100%: $H_2O/H_2SO_4$ | |

Beispiel 4

In der in Beispiel 1 verwendeten Sulfonierungsapparatur werden 400 g 100%ige Schwefelsäure vorgelegt. Zu dieser werden bei 30° C gleichzeitig 225 g (1 Mol) 1-Naphthylamin-7-sulfonsäure (99%ig) und 246 g 65%iges Oleum ( = 2 Mol SO₃) innerhalb von 30 Minuten eingetragen. Anschließend wird das Sulfonierungsgemisch etwa 8 Stunden bei 30° C nachgerührt. Anschließend wird das Sulfonierungsgemisch in 1400 g Wasser eingetragen und — wie in Beispiel 1 beschrieben — aufgearbeitet.

Es werden 504 g 1-Naphthylamin-4.7-disulfonsäure in Form eines schwach grauen, schwefelsäurefeuchten Produktes erhalten. Die Ausbeute an 1-Naphthylamin-4.7-disulfonsäure beträgt 90,7% der Theorie bezogen auf eingesetzte 1-Naphthylamin-7-sulfonsäure.

Die Hochdruck-Flüssigkeitschromatographie des schwefelsäurefeuchten Produktes ergab folgende Zusammensetzung des Produktes:

| | |
|---|---|
| 1-Naphthylamin-4.7-disulfonsäure | 54,9 Gew.-% |
| 1-Naphthylamin-7-sulfonsäure | 0,05 Gew.-% |
| 1-Naphthylamin-2.4.7-trisulfonsäure | 0,05 Gew.-% |
| unbekannte Verunreinigungen (6-Komponenten) | 1,5 Gew.-% |
| Reste bis 100%: $H_2O/H_2SO_4$ | |

Beispiel 5
(Sulfonierung gemäß GB-PS 15 223)

In der in Beispiel 1 verwendeten Sulfonierungsapparatur werden 780 g 25%iges Oleum ( = 2,44 Mol SO₃) vorgelegt. In dieses werden bei 20° C innerhalb von 45 Minuten 225 g (1 Mol) 1-Naphthylamin-7-sulfonsäure (99%ig) eingetragen. Die Sulfonierungsmischung wird anschließend 3 Stunden auf 50° C erwärmt.

Die durch Hochdruckflüssigkeitschromatographie des fertigen Sulfonierungsproduktes bestimmte Ausbeute an 1-Naphthylamin-4,7-disulfonsäure beträgt 55% der Theorie bezogen auf eingesetzte 1-Naphthylamin-7-sulfonsäure.

Die Hochdruck-Flüssigkeitschromatographie des Sulfonierungsgemisches ergab folgende Zusammensetzung:

| | |
|---|---|
| 1-Naphthylamin-4.7-disulfonsäure | 16,6 Gew.-% ( = 55 Mol-%) |
| 1-Naphthylamin-3.7-disulfonsäure | 0,35 Gew.-% ( = 1,1 Mol-%) |
| 1-Naphthylamin-5.7-disulfonsäure | 0,13 Gew.-% ( = 0,4 Mol-%) |
| 1-Naphthylamin-2.4.7-trisulfonsäure | 18,8 Gew.-% ( = 28,3 Mol-%) |

Beispiel 6
(Sulfonierung gemäß Donaldson, Seite 203)

In der in Beispiel 1 beschriebenen Sulfonierungsapparatur werden 713 g 100%ige Schwefelsäure vorgelegt. In diese werden 225 g (1 Mol) 1-Naphthylamin-7-Sulfonsäure (99%ig) bei einer Temperatur von höchstens 40° C eingetragen. Dann werden bei einer Temperatur von höchstens 35° C innerhalb einer Stunde 580 g 65%iges Oleum ( = 4,7 Mol SO₃) zugetropft. Das Sulfonierungsgemisch wird etwa 14 Stunden bei 20° C nachgerührt. Anschließend wird es in kaltes Wasser eingetragen. Durch Kühlen wird dafür gesorgt, daß keine Überhitzung eintritt. Nach dem Eintragen wird die erhaltene wäßrige Lösung des Sulfonierungsgemisches auf 60° C erwärmt, mit 368 g Kochsalz versetzt und anschließend

4

unter Rühren wieder auf 20° C abgekühlt. Der ausgefallene Niederschlag wird abgesaugt und so lange mit gesättigter Kochsalzlösung gewaschen, bis die ablaufende Lösung fast farblos ist.

Es werden 470 g 1-Naphthylamin-4.7-disulfonsäure-mononatriumsalz in Form eines schwach gefärbten Produktes erhalten. Die Ausbeute an 1-Naphthylamin-4.7-disulfonsäure-mononatriumsalz beträgt 84% der Theorie, bezogen auf 1-Naphthylamin-7-sulfonsäure.

Die Hochdruck-Flüssigkeitschromatographie des schwefelsäurefeuchten Produktes ergab folgende Zusammensetzung des Produktes:

| | |
|---|---|
| 1-Naphthylamin-4.7-disulfonsäure | 54,2 Gew.-% |
| 1-Naphthylamin-7-sulfonsäure | 0,05 Gew.-% |
| 1-Naphthylamin-2.4.7-trisulfonsäure | 0,3 Gew.-% |
| unbekannte Verunreinigungen | 1,5 Gew.-% |
| Rest bis 100%: $H_2O/NaCl$ | |

## Beispiel 7

Es wird wie in Beispiel 1 beschrieben sulfoniert.

Dann wird das Sulfonierungsgemisch so auf 2650 g Wasser ausgetragen, daß die Temperatur dabei auf 80 bis 90° C ansteigt. Nun tropft man vorsichtig 500 g 50%ige Natronlauge zu. Die Temperatur der Suspension darf dabei bis auf ca. 100° C ansteigen. Anschließend wird unter Rühren langsam auf 20° C abgekühlt (Abkühlzeit 5 bis 6 Stunden). Der Niederschlag wird abgesaugt.

Es werden 488 g 1-Naphthylamin-4,7-disulfonsäuremononatriumsalz in Form eines schwach grau gefärbten Produktes erhalten. Die Ausbeute an 1-Naphthylamin-4.7-disulfonsäure-mononatriumsalz beträgt 91,3% der Theorie, bezogen auf 1-Naphthylamin-7-sulfonsäure.

Die Hochdruck-Flüssigkeitschromatographie des schwefelsäurefeuchten Produkts ergab folgende Zusammensetzung des Produktes:

| | |
|---|---|
| 1-Naphthylamin-4.7-disulfonsäure | 56,8 Gew.-% |
| 1-Naphthylamin-7-sulfonsäure | 0,05 Gew.-% |
| 1-Naphthylamin-2.4.7-trisulfonsäure | Spur |
| unbekannte Verunreinigungen (5 Komponenten) | 1,0 Gew.-% |
| Rest bis 100%: $H_2O/Na_2SO_4$ | |

## Patentansprüche

1. Verfahren zur Herstellung von 1-Naphthylamin-4.7-disulfonsäure durch Sulfonieren von 1-Naphthylamin-7-sulfonsäure mit Oleum, Aufarbeiten des Sulfonierungsgemisches durch Eintragen in Wasser und Isolieren der 1-Naphthylamin-4-7-disulfonsäure, dadurch gekennzeichnet, daß man das Oleum bei einer Temperatur von 10 bis 70° C entweder gleichzeitig mit der 1-Naphthylamin-7-sulfonsäure zu vorgelegter Schwefelsäure gibt oder zu in Schwefelsäure gelöster bzw. suspendierter 1-Naphthylamin-7-sulfonsäure gibt und ein solches Molverhältnis Schwefeltrioxid : 1-Naphthylamin-7-sulfonsäure anwendet, daß 1,2 bis 3 Mol Schwefeltrioxid auf 1 Mol 1-Naphthylamin-7-sulfonsäure entfallen.

2. Verfahren gemäß Anspruch 1, dadurch gekennzeichnet, daß man ein solches Molverhältnis Schwefeltrioxid zu 1-Naphthylamin-7-sulfonsäure anwendet, das 1,75 bis 2,5 Mol Schwefeltrioxid auf 1 Mol 1-Naphthylamin-7-sulfonsäure entfallen.

3. Verfahren gemäß Anspruch 1 und 2, dadurch gekennzeichnet, daß man zum Vorlegen bzw. Lösen bzw. Suspendieren 80 bis 100%ige Schwefelsäure verwendet.

4. Verfahren gemäß Anspruch 1 bis 3, dadurch gekennzeichnet, daß man die zum Vorlegen bzw. Lösen, bzw. Suspendieren verwendete 80 bis 100%ige Schwefelsäure in einer solchen Menge anwendet, daß auf 1 Mol 1-Naphthylamin-7-sulfonsäure 6 bis 8 Mol Schwefelsäure entfallen.

## Claims

1. Process for the preparation of 1-naphthylamine-4,7-disulphonic acid by sulphonating 1-naphthylamine-7-sulphonic acid with oleum, working up the sulphonation mixture by introducing it into water, and isolating the 1-naphthylamine-4,7-disulphonic acid, characterized in that the oleum is added at a temperature from 10 to 70° C, either simultaneously with the 1-naphthylamine-7-sulphonic acid to initially introduced sulphuric acid, or to the 1-naphthylamine-7-sulphonic acid, which is dissolved or suspended in sulphuric acid, and such a molar ratio of sulphur trioxide : 1-naphthylamine-7-sulphonic acid is used that 1.2 to 3 mols of sulphur trioxide are present per mol of 1-naphthylamine-7-sulphonic acid.

2. Process according to claim 1, characterized in that such a molar ratio of sulphur trioxide to 1-naphthylamine-7-sulphonic acid is used that 1.75 to 2.5 mols of sulphur trioxide are present per mol of 1-naphthylamine-7-sulphonic acid.

3. Process according to claim 1 and 2, characterized in that 80 to 100% strength sulphuric acid is used as the component initially introduced or for obtaining the solution or suspension.

4. Process according to claim 1 to 3, characterized in that the 80 to 100% strength sulphuric acid used as the component initially introduced or for obtaining the solution or suspension is used in such an amount that 6 to 8 mols of sulphuric acid are present per mol of 1-naphthylamine-7-sulphonic acid.

## Revendications

1. Procédé pour la fabrication d'acide 1-naphtylamine-4,7-disulfonique par sulfonation de l'acide 1-naphtylamine-7-sulfonique par l'oléum, traitement du mélange de sulfonation par introduction d'eau et isolement de l'acide 1-naphtylamine-4.7-disulfonique, caractérisé en ce que l'on ajoute l'oléum soit simultanément avec l'acide 1-naphtylamine-7-sulfonique à l'acide sulfurique préalablement chargé, soit à l'acide 1-naphtylamine-7-sulfonique dissous ou en suspension dans l'acide sulfurique, à une température de 10−70°C et l'on applique un rapport molaire trioxyde de soufre/acide 1-naphtylamine-7-sulfonique tel qu'il y ait 1, 2 à 3 moles de trioxyde de soufre pour 1 mole d'acide 1-naphtylamine-7-sulfonique.

2. Procédé selon la revendication 1, caractérisé en ce que l'on applique un rapport molaire trioxyde de soufre/acide 1-naphtylamine-7-sulfonique tel qu'il y ait 1,75 à 2,5 moles de trioxyde de soufre pour 1 mole d'acide 1-naphtylamine-7-sulfonique.

3. Procédé selon la revendication 1 ou 2, caractérisé en ce que l'on utilise pour la charge, ou la mise en solution ou en suspension de l'acide sulfurique à 80−100%.

4. Procédé selon les revendications 1 à 3, caractérisé en ce que l'on applique l'acide sulfurique à 80−100%, utilisé pour la charge ou la mise en solution ou en suspension, en quantité telle qu'il y ait 6 à 8 moles d'acide sulfurique pour 1 mole d'acide 1-naphtylamine-7-sulfonique.